# EUROPEAN PATENT APPLICATION

(11) **EP 2 535 087 A2**
(43) Date of publication of application: **19.12.2012**
(21) Application number: 12160474.8
(22) Date of filing: 21.03.2012
(51) Int. Cl.: A61Q 19/00, A61K 8/81, A61K 8/87, A61K 8/73, A61K 8/368, A61K 8/49, A61K 8/365, A61K 8/34, A61K 8/60, A61K 8/35

(54) **Cosmetic, pharmaceutical or nutraceutical formulations containing antioxidant molecules and polymeric materials**

(30) Priority: 21.03.2011 IT CS20110009
(71) Applicant: Universita' Della Calabria, 87036 Rende (CS) (IT)
(72) Inventor: Puoci, Francesco, 87040 Castrolibero (CS) (IT); Iemma, Francesca, 87036 Rende (IT); Pezzi, Vincenzo, 87036 Rende (IT); Picci, Nevio, 87036 Rende (IT); Cirillo, Giuseppe, 87056 S.Stefano di Rogliano (CS) (IT); Curcio, Manuela, 87100 Cosenza (IT); Parisi, Ortensia Ilaria, 87040 Castrolibero (CS) (IT)
(74) Representative: Perrotta, Aldo

(57) **Abstract**

The knowledge of the damage from chronic exposure to exogenous factors, such as sunlight and oxidizing agents is increasing, thanks to the information campaigns. The protective action of these factors must pay attention to the issues of toxicity resulting from topical formulations which should provide specific requirements, including stability and safety.

In this regards, particular attention is focused on sunscreens, especially on the organic ones, which can show photostability and toxicity concerns. The importance of the filters toxicity concerns has been greatly exacerbated in recent years to the point that the producers of raw materials and technical formulators have implemented various strategies in order to develop stable and safe sunscreens. In this context, the use of "SPF boosters", raw materials are able to increase the SPF of a formulation of the sun exploiting different mechanisms of action, is an interesting approach in the development of solar products.

Considerable interest has also led to the preparation of formulations for the protection from oxidizing agents (ozone, hypochlorite and environmental pollutants) responsible for a pathological condition, such as oxidative stress, which can damage different cell structures. Oxidative stress, indeed, involves a degradation of cells and tissues with a loose in their efficiency. The premature aging of the skin is one of the most popular signs.

This idea is based on the use of polyvinylpyrrolidone (PVP) functionalized with antioxidant molecules such as rosmarinic acid, ellagic acid, epigallocatechin gallate, resveratrol, hydroxytyrosol, to be used as boosters of SPF and / or protective agents for skin and hair from exogenous factors such as harmful UV radiation, ozone and chemical oxidants. These antioxidant-polymer conjugates represent a breakthrough in the world of raw and semi-processed materials in the cosmetic, pharmaceutical and nutraceutical markets.

At present, polymeric and antioxidant compounds, such as polyphenols, are individually used in cosmetic, pharmaceutical and nutraceutical formulations, but no examples of antioxidant-polymer conjugates are reported.

## Description

The awareness in the population of the damages resulting from chronic exposure to exogenous agents, such as oxidizing agents and solar radiations, is increasing, also thanks to public information campaigns. The protection against the damages produced by these factors shifts the attention on the toxicity problems resulting from topical application of specific formulations which should guarantee numerous requirements, including stability and tolerability ones. In this context, particular attention is given to sunscreens, especially the organic ones, which can present problems of photo-stability and toxicity. In recent years, the general attention towards the toxicity problems of these formulations has been greatly exacerbated, and both producers of raw materials and formulators have implemented various strategies in order to develop stable and reliable solar products. In this context, the use of the so-called "boosters of SPF", i.e. raw materials able to enhance the SPF of a sunscreen, represents an interesting approach for the development of sun protection products. Considerable interest has also attracted the design of formulations for the protection from the oxidizing agents (ozone, hypochlorite and environmental pollutants) responsible for a pathological condition, the oxidative stress, which can cause severe damages to different cell compartments. Oxidative stress, indeed, determines the aging of the cells and, therefore, of the tissues, leading to different pathological states, e.g. premature skin aging.

This patent idea is focused on the use of polyvinylpyrrolidones (PVP) functionalized with antioxidant molecules, such as rosmarinic and ellagic acids, epigallocatechin-3-gallate, resveratrol, hydroxytyrosol, to be used as SPF boosters and/or protective agents against the damages caused by UV radiations, ozone and oxidant chemical agents on skin and hair. These polymer-antioxidant conjugates represent an innovation in the world of raw materials and semi-finished products used in cosmetic, pharmaceutical and nutraceutical market. In the formulations currently available on the market, indeed, both polymeric materials and antioxidant substances, such as polyphenols, are individually widely used, but to date, no examples of antioxidant-polymer conjugates are reported.

### Technical Field of the Invention

This invention is about a cosmetic, pharmaceutical or nutraceutical formulation containing antioxidant molecules covalently linked to polymeric materials.

### State of Art

Polymeric materials are currently employed in the preparation of protecting formulations against the damages caused by different exogenous agents, such oxidant agents and/or UV radiations (SPF boosters). For example, in US6495123 and in US 7097828 the use of insoluble polymers and polyurethane systems in sunscreen is documented. Among the soluble polymers, acrylates (US 20070264204), styrene/acrylates composities (US 20090202459) and polyvinylpyrrolidones are the most widely employed. CA1046414 reports on the use of polymeric materials in cosmetic formulations able to maintain the natural skin hydration; WO03066791 is about the development of polymeric materials with film-forming activity acting as both skin and hair protecting agents against the damages induced by UV radiations and drug delvery devices. DE 102004014612 describes cosmetic and dermatological formulations containing taurin and polymers such as nylon, PVP, starch and cellulose derivatives to be used as skin protecting and hydrating agents, while GB 1487441 demonstrates that polymers characterized by the presence of carboxylic and hydroxyl groups allow to preserve the skin hydration. US 6436376 reports on the use of Tricontanyl PVP as SPF booster and US 6436377 on the employment of an interpenetrated polymer based on PVP/dimethiconylacrylate/polycarbamyl/polyglycol ester as SPF-enhanced UV-photoprotection of human skin, hair and/or scalp. There are also examples of functionalization of PVP with bioactive molecules such as heparin (US 4239664), but no example of polymeric materials functionalized with antioxidant molecules are reported, although several scientific data document the protecting activity exerted by such molecules against the damages caused by UV radiation and environmental pollutants. In this regards, US 20070224138 and US 20040170581 describe the use of Ginkgo Biloba extracts and caffeic acid and its derivatives, respectively, as sunscreen boosters, and US 4517175 reports on the employment of flavanols as gloss- and softness-enhancing agents. Finally, although both polymeric materials and antioxidant molecules are commonly used in cosmetic formulations, to date no examples of formulations containing polymeric materials functionalized with such molecules are reported.

### Technical Problems

A first problem is related to instability of antioxidant molecules inside the cosmetic formulations, in particular in cosmetic formulations possessing a protective activity against the damages produced by exogenous factors, such as oxidant agents, pollutants and UV radiations. For example, the strong chemical activation energy derived from ultraviolet radiation induces hydrolytic reactions, with a consequent reduction or loss of the antioxidant, filtering and protecting activities. A reduction of UVB filtering capacity would lead to a reduction in SPF of the formulation, and therefore to a higher burn risk, while a reduced capacity to filter UVA radiation might go unnoticed, exposing to a greater risk of adverse chronic effects, which are characteristic of these bands. Furthermore, the photo-degradation of the sunscreen molecule generates potentially harmful chemical substructures which can induce allergic sensitization processes, skin irritation or toxicity phenomena due to their trans-dermal absorption. A second problem is related to the necessity to confer antioxidant and photo-protecting activity to polymeric materials.

This kind of materials, indeed, are employed in pharmaceutical and cosmetic formulations as excipients, in order to modulate and optimize their rheological and chemical-physical properties, but they do not possess any protecting activity against oxidative stress. Then, the covalent linkage of an antioxidant molecule to a polymeric material provides it a high added value.

### Solutions to the technical problem

The present patent is specifically devoted to the resolution of the technical problem related to the low stability of antioxidant molecules in cosmetic, pharmaceutical and nutraceutical formulations through the covalent conjugation with polymeric materials.

As a result of the covalent coupling of an antioxidant to a polymer, an increase of the stability of the antioxidant in obtained, while its activity is retained. On the others hands, this chemical modification allows conferring a high value to the polymeric materials. Thus, by using an antioxidant-polymer conjugate, the stability of the antioxidant is raised by preserving its activity.

### Working

The polymeric materials form a film coating the surface of the hair (if used in the "hair-care") or of the skin (when used in skin care), shielding the UV radiation and carrying out a protective action against damage caused by others exogenous oxidizing agents, air pollutants, etc. Among the different polymeric materials, Polyvinylpyrrolidone (PVP) is the material of choice due to its stability and its film-forming properties which guarantee a better adhesion to the skin and/or to the hair. Furthermore, the antiinflammatory and antioxidant properties of polyphenols and their ability to absorb UV radiation enhancing the SPF of a formulation solar are well known; these molecules are, however, relatively unstable as they may undergo auto-oxidation phenomena induced by the same UV radiation, by heating or oxidizing agents. The whole of these considerations led to the present idea. The conjugation of phenols with polymeric materials significantly increases the stability of antioxidants giving a high value and greater efficiency to the final materials. The conjugation to the polymer also reduces the phenomena leading to skin absorption of antioxidant into the systemic circulation with potential acute and chronic side effects.

### Industrial application

Excipients for cosmetic formulations, excipients for liquid, solid and semi-solid pharmaceutical forms, components of nutraceutical formulations.

### Description of one or more embodiments

The conjugated polymer-antioxidant, and in particular the conjugates between PVP and gallic acid, ellagic acid, ferulic acid, caffeic acid, cinnamic acid, catechin, quercetin, epigallocatechin gallate, hydroxytyrosol, rosmarinic acid, oleuropein, curcumin, and resveratrol can be used, at different concentrations, in the formulation of shampoos, conditioners, creams, pastes, ointments, gels, foams, masks, oils, but also products for nutraceutical use.

### Advantage

The use of polymeric materials functionalized with antioxidant molecules allows combining the advantages of both polymeric materials and antioxidants. In particular: Lower degradability of the polymeric material: the conjugation of a polymer system with antioxidant molecules confers to the conjugate greater stability in comparison to the starting material, leading to a reduction of the degradation reactions.

Greater stability of the antioxidant molecule: the presence of a covalent bond avoids the phenomenon of migration and extrusion of the antioxidant molecule from the formulation, which that are typical of formulations in which the antioxidant is simply mixed with the other ingredients of the preparation.

Increased safety: the conjugation of the antioxidant molecule to the polymeric material reduces the toxic effects related to processes of absorption through the skin that can result in various phenomena including the accumulation in the tissues.

Speed of preparation: related to a high versatile synthetic strategy.

Easy industrial scale-up: related to a synthetic procedure allowing to operate in non-drastic conditions.

### Variants:

Several antioxidant-polymer conjugates can be used as excipients for cosmetic, pharmaceutical and nutraceutical formulations. Among the polymeric materials that can be used for the preparation of conjugates with antioxidant activity the most representative are: PVP, chitosan, alginate, inulin, starch, dextran, polymethacrylic acids, polymethyl methacrylates, polyacrylamides, polyurethanes, and polystyrenes; while as antioxidant can be used: gallic acid, ellagic acid acid, ferulic acid, caffeic acid, cinnamic, catechin, quercetin, epigallocatechin trigallate, hydroxytyrosol, rosmarinic acid, oleuropein, curcumin, and resveratrol. All the obtained materials are effective in protecting against UV irradiation and oxidative degradation reactions. The following examples further illustrate, but not limited, the invention. These examples refer to the composition of cosmetic formulations, in particular a cosmetic cream and a shampoo, containing two different antioxidant-PVP conjugates, PVP-resveratrol and the PVP-rosmarinic acid, respectively. In the table the excipients necessary for the preparation of the two formulations are also reported.

### Example 1.

| Cosmetic cream | |
|---|---|
| PVP-Resveratrol | 1.5 % |
| Sepigel | 1.5 % |
| Glycerine | 3% |
| Almond Oil | 6% |
| Microcombin | 1 % |
| Perfume | 0.3% |
| Water to 100 g | |

### Example 2.

| Shampoo | |
|---|---|
| PVP-Rosmarinic acid | 0.3 % |
| Sodium laureth sulphate | 25 % |
| Cocamide propyl betaine | 12% |
| Cocamide dea | 2.5% |
| Citric Acid | 0.7 % |
| Polyquaternium 7/39 | 3% |
| Acnibio | 0.01 % |
| Perfume | 1% |
| PEG 40 | 3% |
| Water to 100 g | |

### Figure Captions

Figure 1 shows the insertion of a covalent antioxidant molecule into a polymer chain which can be realized through the use of different strategies. The figure is a general scheme which provides for the grafting induced by free radicals in which A is the antioxidant molecule.
Figure 2 represents the protective action of a balm containing the PVP - rosmarinic acid conjugate at a concentration of 0.5% by weight on hair subjected to the action of oxidizing agents. a) Hair treated with sodium hypochlorite; b) hair pretreated with the conjugate and exposed to the same oxidizing agent

## Claims

1. Cosmetic, pharmaceutical or nutraceutical formulations containing antioxidant molecules and polymeric materials **characterized by** the fact that the antioxidant molecules are covalently bounded to the polymeric chains.

2. Cosmetic, pharmaceutical or nutraceutical formulation according to claim 1, **characterized by** the fact that said polymeric materials are polymers such as PVP, chitosan, alginate, inulin, starch,dextran, poly methacrylic acids, poly methylmetacrilates, polyacrylamides, polyuretanes,polystyrenes.

3. Cosmetic, pharmaceutical or nutraceutical formulation according to claim 1 or 2, **characterized by** the fact that said antioxidant molecules are molecules such as gallic acid, ellagic acid,ferulic acid, caffeic acid, cinnamic acid, catechin, quercetin, epigallocatechingallate,hydroxytyrosol, rosmarinic acid, oleuropein, curcumin, resveratrol.

4. Cosmetic formulation according to claims 1, 2 and 3 **characterized by** the fact that said formulation is in the form of cream having the composition constituted by polyvinyl pyrrolidone-resveratrol, Sepigel, Glycerine, Almond oil, Paraben mix, perfume and water in which the polyvinyl pyrrolidone-resveratrol conjugate is added in percentage of 1.5

5. Cosmetic formulation according to claims 1, 2 and 3 **characterized by** the fact that said formulation is in the form of shampoo having the composition constituted by polyvinyl pyrrolidone-rosmarinic acid, Sodium laureth sulphate, Cocamide propyl betaine, Cocamide dea, Citric acid, Polyquaternium 7/39, biodegradable preservative and fungicide, perfume, hydrogenated castor oil and water, in which the polyvinyl pyrrolidone-rosmarinic acid conjugate is added in percentage of 0.3.
